(19) Europäisches Patentamt / European Patent Office / Office européen des brevets

(11) **EP 3 090 801 B1**

(12) **EUROPEAN PATENT SPECIFICATION**

(45) Date of publication and mention of the grant of the patent:
**25.07.2018 Bulletin 2018/30**

(51) Int Cl.:
*B01J 23/89* (2006.01)  *C07C 11/167* (2006.01)
*B01J 37/18* (2006.01)  *B01J 37/34* (2006.01)
*B01J 21/14* (2006.01)  *B01J 35/00* (2006.01)
*B01J 35/02* (2006.01)  *B01J 35/10* (2006.01)
*B01J 37/02* (2006.01)

(21) Application number: **15001396.9**

(22) Date of filing: **08.05.2015**

(54) **CATALYST FOR 1,3-BUTADIENE PRODUCTION FROM ETHANOL**

KATALYSATOR ZUR 1,3-BUTADIEN-HERSTELLUNG AUS ETHANOL

CATALYSEUR POUR PRODUCTION DE 1,3-BUTADIÈNE À PARTIR D'ÉTHANOL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**09.11.2016 Bulletin 2016/45**

(73) Proprietor: **The Siam Cement Public Company Limited**
**10800 Bangkok (TH)**

(72) Inventors:
• **Sae-Khow, Ornthida**
**10800 Bangkok (TH)**
• **Bussayajarn, Narissara**
**10800 Bangkok (TH)**
• **Totong, Sansanee**
**10140 Bangkok (TH)**
• **Laosiripojana, Navadol**
**10140 Bangkok (TH)**
• **Tripathi, Anamol**
**10140 Bangkok (TH)**

(74) Representative: **Kador & Partner PartG mbB**
**Corneliusstraße 15**
**80469 München (DE)**

(56) References cited:
**US-A- 4 101 451**   **US-A1- 2003 171 629**
**US-A1- 2013 123 554**

• **MAKSHINA E V ET AL:** "Catalytic study of the conversion of ethanol into 1,3-butadiene", **CATALYSIS TODAY, vol. 198, no. 1, 21 June 2012 (2012-06-21), pages 338-344, XP028958465, ISSN: 0920-5861, DOI: 10.1016/J.CATTOD.2012.05.031**
• **LARINA OLGA V ET AL:** "Ethanol Conversion to 1,3-Butadiene on ZnO/MgO-SiO2Catalysts: Effect of ZnO Content and MgO:SiO2Ratio", **CATALYSIS LETTERS, SPRINGER NEW YORK LLC, UNITED STATES, vol. 145, no. 5, 13 March 2015 (2015-03-13), pages 1162-1168, XP035499902, ISSN: 1011-372X, DOI: 10.1007/S10562-015-1509-4 [retrieved on 2015-03-13]**
• **B. B. CORSON ET AL:** "Butadiene from Ethyl Alcohol. Catalysis in the One-and Two-Stop Processes.", **INDUSTRIAL & ENGINEERING CHEMISTRY, vol. 42, no. 2, 1 February 1950 (1950-02-01), pages 359-373, XP55051002, ISSN: 0019-7866, DOI: 10.1021/ie50482a039**

**Description**

The present invention relates to catalysts for the conversion of ethanol to 1,3-butdiene comprising at least two transition metal elements.

1,3-butadiene is an important starting material for the chemical industry, especially the polymer industry. About 60% of the world production of 1,3-butadiene is used in the preparation of synthetic rubber. Further commercially important polymers made from 1,3-butadiene are styrene-butadiene rubbers, polybutadiene, styrene-butadiene latex, and acrylonitrile-butadiene-styrene polymer. The relatively recent exploitation of shale gas has resulted in 1,3-butadiene scarcity, since natural gas chemical feedstock has fewer $C_4$-hydrocarbons than oil feedstock used in petrochemical production.

The demand growth for tires and polymers from emerging economies and the volatility of the natural rubber market will continue to drive efforts in renewable butadiene. Thus, currently a lot of attention is paid to the usage of such alternative and renewable resources for fuels and chemicals. Due to sustainable development, which is based on non-petroleum feedstock, ethanol is one of the most relevant potential sources of "bio"-carbon today.

Catalytic conversion of ethanol into 1,3-butadiene is an old industrially proven route. From the 1920s to the early 1960s, ethanol was converted to 1,3-butadiene, hydrogen and water at 400 to 450 °C in a one-step process with yields up to 72 mol-%. The mechanism of the ethanol to 1,3-butadiene transformation is extremely complicated and is still subject of debate. However, the following five steps are most likely involved:

(i) ethanol transformation to acetaldehyde and hydrogen;

(ii) aldol condensation of acetaldehyde yielding acetaldol;

(iii) dehydration of acetaldol yielding crotonaldehyde;

(iv) reaction between crotonaldehyde and ethanol yielding crotyl alcohol and acetaldehyde (Meerwein-Ponndorf-Verley);

(v) dehydration of crotyl alcohol yielding 1,3-butadiene.

Gas-phase synthesis of butadiene from ethanol or a mixture of ethanol and acetaldehyde in the presence of catalysts is known in the prior art (see e.g. WO 2012/015 340 A1). However, these catalysts comprise a metal chosen from the group of silver, gold or copper, and metal oxide chosen from the group of magnesium, titanium, zirconium, tantalum or niobium oxides.

WO 2012/015 340 A1 discloses a method utilizing a condensation process under the conditions of continuous flow fixed bed reactor. The catalyst was designed to reach high yield and selectivity as regards 1,3-butadiene and high level of conversion of the feed. Disadvantages of these catalysts are that they show only medium stability in the reaction, and the high price of the used metals.

Prior art document KR 2014/050 531 A discloses a method for the production of a catalyst for the conversion of ethanol to 1,3-butadiene. In this method, a transition-metal oxide chosen from group III, group IV and group V metal oxides, preferably hafnium oxide, zirconium oxide, tantalum oxide, zinc oxide and niobium oxide is supported on mesoporous silica. These catalysts, even if enhanced by the larger surface of the silica, suffer from similar disadvantages as those described in WO 2012/015 340 A1.

The catalytic conversion of ethanol to 1,3-butadiene has been studied in 'Makshina, W. Janssens,B.F. Sels, P.A. Jacobs, (2012) "Catalytic study of the conversion of ethanol into 1,3-butadiene", Catalysis Today, 198, 338-344'. In this study, catalysts have been used comprising a magnesia-silica support being doped with single transition metal(oxide)s.

In view of the general importance of the conversion of ethanol to 1,3-butadiene as outlined above, there is still the need to improve the stability of catalysts used in this reaction at comparable or even better yields of 1,3-butadiene and selectivity towards 1,3-butadiene compared to prior art catalysts.

Furthermore, given the high prices of catalytically active metals, there is also the need for finding catalysts which are significantly less costly in production than the catalysts mentioned in the prior art.

Thus, it is an object of the present invention to find a catalyst for the conversion of ethanol to 1,3-butadiene, which shows minimal degradation (maximum stability) during catalysis at acceptable or even superior turnover numbers and 1,3-butadiene selectivity.

Furthermore, it is an object of the present invention to provide a catalyst achieving the mentioned objects which can be provided in a less costly manner.

The present invention is based on the finding that the above object can be achieved by using a catalyst for the conversion of ethanol to 1,3-butadiene according to claim 1.

Therefore, the present invention provides a catalyst for the conversion of ethanol to 1,3-butadiene according to claim 1.

It has been surprisingly found that the combination of the two metals silver (Ag) and copper (Cu) in weight ratio between silver (Ag) and copper (Cu) in the range of 10:1 and 1:10 on a support comprising as a first metal oxide silica and as a second metal oxide magnesium oxide results in a more stable catalyst and hence in longer lifetime cycles of the catalyst during the reaction. In particular, it has been surprisingly found that the combination of the silver (Ag) and the copper (Cu) metal on the support shows a synergistic stabilization effect.

At the same time, the performance and the selectivity of the catalyst is maintained at an acceptable, high level, and, finally, the catalyst does not comprise high-cost elements so that it can be produced at comparatively low cost.

The term "conversion" such as used herein represents the amount of ethanol used during reaction divided by the amount of ethanol fed to the reaction.

The term "selectivity" such as used herein represents the amount of 1,3-butadiene produced during reaction divided by the total amount of all products of the reaction.

The term "yield" such as used herein represents the amount of 1,3-butadiene produced during reaction divided by the amount of ethanol fed to the reaction.

Furthermore, the terms "stabilization" or "stability" are related to the deactivation of the catalyst. A catalyst is naturally deactivated during the reaction. This deactivation is calculated as the percentage by which the initial yield of the catalyst has been reduced at the time of the measurement of the deactivation. The lower the deactivation, which is present in a catalyst, the better the stabilization of such a catalyst.

The term "metal form" denotes that Ag and Cu are, at least in part, present on the support in their oxidation state 0. Preferably, all of the Ag and/or Cu present on the catalyst support is in metal form.

The term "calcination" as used herein denotes a thermal treatment process in the presence of air or oxygen applied to ores and other solid materials to bring about a thermal decomposition, phase transition, or removal of a volatile fraction. This process normally takes place at temperatures below the melting point of the product materials.

The support according to the present invention preferably comprises a first metal oxide.

Preferably, the first metal oxide is silica fume.

Preferably, the second metal oxide is nano-sized magnesium oxide.

Preferably the first and the second metal oxide are present in the support in a weight ratio in the range of 100:1 to 1:100, preferably of 80:1 to 1:80, more preferably of 40:1 to 1:40, and most preferably of 10:1 to 1:10.

Still more preferably, the first and the second metal oxide are present in the support in a weight ratio in the range of 1:1 to 1:5, preferably 1:1.5 to 1:4, and most preferably 1:1.7 to 1:3.

In a preferred embodiment, the catalyst of the present invention comprises Ag and Cu in metal form in a weight ratio in the range of 5:1 and 1:5 and more preferably of 3:1 and 1:3.

Still more preferably, the catalyst comprises Ag and Cu in metal form in a weight ratio in the range of 2:1 and 1:2, more preferably of 1.5:1 and 1:1.5, and most preferably of 1.1:1 and 1:1.1.

Furthermore, the catalyst according to the present invention preferably has a particle size between 1 and 100 $\mu$m, preferably 5 and 80 $\mu$m, and most preferably 10 and 60 $\mu$m, measured by electron microscopy (SEM) according to ASTM standard E986:04.

Still more preferably, the catalyst according to the present invention has a particle size between 15 and 40 $\mu$m, more preferably 17 and 35 $\mu$m, and most preferably 20 and 30 $\mu$m, measured by electron microscopy (SEM) according to ASTM standard E986:04.

In a preferred embodiment, the combined weight of Ag and Cu in metal form on the catalyst, also denoted as "metal loading", is in the range of 1% and 30%, preferably of 2% and 25%, and most preferably of 3% and 21%, measured according to by X-ray fluorescence (XRF) techniques according to ASTM standard D4326:04.

In a further preferred embodiment of the invention, the catalyst has a surface area of between 60 to 400 m$^2$/g, preferably 100 to 350 m$^2$/g, and most preferably 150 to 300 m$^2$/g, measured by Brunauer-Emmett-Teller method (BET) according to ASTM standard D6556:10.

The catalyst according to the present invention preferably has a reduction temperature of 200 to 280 °C, more preferably of 210 to 270 °C, still more preferably of 220 to 265 °C, still more preferably of 230 to 260 °C, and most preferably of 240 to 250 °C, determined by temperature-programmed reduction (TPR).

The reduction temperature of the catalyst is the temperature at which the precursors of Ag and Cu, which usually are Ag and Cu salts, and preferably are selected from independently Ag and Cu chlorides and/or nitrates, are transformed into Ag and Cu in metal form.

Finally, the metal dispersion of the catalyst of the present invention is preferably 2% to 20%, more preferably 4% to 15%, and most preferably 5% to 12%, measured by volumetric hydrogen chemisorption.

The present invention further provides a method for preparing a catalyst for the conversion of ethanol to 1,3-butadiene comprising the steps of

a) calcining a support comprising as a first metal oxide silica and as a second metal oxide magnesium oxide;

b) impregnating the calcined support with precursors of silver (Ag) and copper (Cu) metal ; and

c) reducing the impregnated catalyst precursor yielding the catalyst so that silver (Ag) and copper (Cu) in metal form are present on the support,

wherein the method prior to step a) of calcining a support comprises the steps of

d) wet-kneading of the first and the second metal oxides to yield an intermediate support,

e) drying the intermediate support.

Calcining step a) preferably is performed at 300 °C to 600 °C, more preferably at 325 °C to 500 °C, and most preferably at 350 °C to 450 °C.

Furthermore, preferably, calcining step a) is performed for 1 to 10 h, more preferably for 2 to 7 h, and most preferably for 3 to 5 h.

Impregnation step b) is preferably performed by incipient wetness impregnation.

Furthermore, impregnation step b) is preferably performed at 30 °C to 120 °C, more preferably at 50 °C to 100 °C, and most preferably at 60 °C to 90 °C.

Still further, impregnation step b) is preferably performed for 1 to 10 h, more preferably for 2 to 7 h, and most preferably for 3 to 5 h.

In impregnation step b) preferably the precursors of silver (Ag) and copper (Cu) metal are silver (Ag) and copper (Cu) compounds, more preferably silver (Ag) and copper (Cu) salts and most preferably, independently, silver (Ag) and copper (Cu) chlorides or silver (Ag) and copper (Cu) nitrates.

In a preferred embodiment, reduction step c) is performed at 300 °C to 600 °C, preferably 325 °C to 500 °C, and most preferably 350 °C to 450 °C. Preferably, reduction step c) is carried out using a hydrogen-containing gas, more preferably using hydrogen.

Preferably, the weight ratio between the first and the second metal oxide in the support is in the range of 100:1 to 1:100, preferably 80:1 to 1:80, and most preferably 40:1 to 1:40. Furthermore, the weight ratio between Ag and Cu on the support is preferably between 5:1 and 1:5 and more preferably between 3:1 and 1:3.

Preferably, the wet-kneading step a') is performed for 2 to 16 h, more preferably for 4 to 14 h, and most preferably for 6 to 10 h.

Furthermore, wet-kneading step a') is preferably performed at 10 °C to 60 °C, more preferably at 15 °C to 40 °C, and most preferably at 18 °C to 24 °C.

In a preferred embodiment, drying step b') is carried out at 30 °C to 200 °C, more preferably at 40 °C to 200 °C, preferably 50 °C to 150 °C, and most preferably 60 °C to 100 °C.

Preferably, the drying step b') is performed for 1 to 15 h, more preferably for 2 to 10 h, and most preferably for 3 to 8 h.

In a first preferred embodiment, the method according to the invention further comprises the following steps after impregnation step b) and preceding reduction step c):

a") filtering the impregnated catalyst precursor;

b") washing the filtered impregnated catalyst precursor;

c") drying the washed catalyst precursor; and

d") calcining the dried catalyst precursor.

Preferably, in washing step b"), the impregnated catalyst precursor is washed, preferably for three times, by deionized water or alcohol, preferably ethanol.

More preferably the impregnated catalyst precursor is washed, preferably for three times, by deionized water and subsequently, preferably three times, by alcohol, preferably ethanol.

Furthermore, drying step c") is carried out preferably at 40 °C to 200 °C, more preferably 50 °C to 150 °C, and most preferably 60 °C to 125 °C.

Preferably, drying step c") is performed for 1 to 15 h, more preferably for 2 to 10 h, and most preferably for 3 to 8 h.

Calcining step d") is preferably carried out for 1 to 10 h, more preferably for 2 to 7 h, and most preferably for 3 to 5 h.

Moreover, calcining step d") is preferably performed at 300 °C to 600 °C, more preferably 325 °C to 500 °C, and most preferably 350 °C to 450 °C.

Furthermore, impregnating step b) preceding step a") is preferably performed for 3 to 4 h at 60 °C to 80 °C.

The metal loading of the Ag and Cu metal on the support of the catalyst obtainable by the method according to the first preferred embodiment as described above is preferably between 1% and 10%, more preferably between 2% and 8%, and most preferably between 3% and 7%, measured by X-ray fluorescence (XRF) techniques according to ASTM standard D4326:04.

Furthermore, the catalyst obtainable by the method of the first preferred embodiment described above has a surface area of preferably 150 to 300 m$^2$/g, more preferably 160 to 270 m$^2$/g, and most preferably 170 to 250 m$^2$/g, measured by Brunauer-Emmett-Teller method (BET) according to ASTM standard D6556:10.

The catalyst obtainable by the method of the first preferred embodiment as described above has a reduction temperature of preferably 200 to 280 °C, more preferably 210 to 270 °C, still more preferably 220 to 265 °C, still more preferably 230 to 260 °C, and most preferably 240 to 250 °C, determined by temperature-programmed reduction (TPR).

Finally, the metal dispersion of the catalyst obtainable by the method of the first preferred embodiment as described above is preferably 5% to 10%, more preferably 5.5% to 9%, and most preferably 6% to 8%, measured by volumetric hydrogen chemisorption.

In a second preferred embodiment, the method according to the invention after impregnation step b) and preceding reduction step c) comprises the following steps:

a") filtering the impregnated catalyst precursor;

b") washing the filtered impregnated catalyst precursor;

c"') microwaving the washed catalyst precursor;

d"') calcining the microwaved catalyst precursor.

Preferably, the microwaving step c"') is performed for 1 to 60 min, more preferably for 2 to 30 min, and most preferably for 3 to 15 min.

Calcining step d"') is performed at 200 °C to 600 °C, preferably 250 °C to 550 °C, and most preferably 300 °C to 500 °C.

Furthermore, impregnating step b) preceding step a"') is preferably performed for 3 to 4 h at 70 °C to 90 °C.

The metal loading of the Ag and Cu metal on the support of the catalyst obtainable by the method of the second preferred embodiment described above is preferably between 5% and 20%, more preferably between 6% and 18%, even more preferably between 7% and 16%, and most preferably between 8 and 12% measured by X-ray fluorescence (XRF) techniques according to ASTM standard D4326:04.

The catalyst obtainable by the method of the second preferred embodiment as described above has a surface area of preferably 150 to 300 m$^2$/g, preferably 160 to 270 m$^2$/g, and most preferably 170 to 250 m$^2$/g, measured by Brunauer-Emmett-Teller method (BET) according to ASTM standard D6556:10.

Furthermore, the catalyst obtainable by the method of the second preferred embodiment as described above has a reduction temperature of the catalyst of preferably 200 to 250 °C, more preferably 210 to 240 °C, and most preferably 215 to 230 °C, determined by Temperature Programmed Reduction (TPR).

Finally, the metal dispersion of the catalyst obtainable by the method of the second preferred embodiment is preferably 5% to 20%, more preferably 7% to 15%, and most preferably 8% to 12%, measured by volumetric hydrogen chemisorption.

The catalyst according to the invention in any of the embodiments as described herein is preferably produced by the method(s) of the invention described in any of the embodiments above.

Furthermore, the present invention pertains to the use of the catalyst in any of the embodiments as described herein for the conversion of ethanol to 1,3-butadiene.

Finally, the present invention provides a process for the catalytic conversion of ethanol to 1,3-butadiene characterized in that said process utilizes the catalyst in any of the embodiments as described herein.

## 1. Measurement methods

*a) Surface area*

The total surface area was determined with a method based on the Brunauer, Emmett, and Teller (B.E.T.) theory of multilayer gas adsorption behavior using multipoint determinations. The described method follows the ASTM standard D6556:10 (Standard Test Method for Carbon Black - Total and External Surface Area by Nitrogen Adsorption).

The surface area and pore size distribution of the catalysts are determined using the Brunauer, Emmett, and Teller (B.E.T.) and the Barrett-Joyner-Halenda (B.J.H.) methods respectively. Nitrogen adsorption-desorption of the catalysts is measured at -196 °C on a Belsorp-max Bel Japan equipment.

The Multipoint Static-Volumetric Gas Adsorption Apparatus Belsorp-max Bel Japan equipment has been used with the following parameters: balance: analytical, with 0.1 mg sensitivity; liquid nitrogen: 98 % or higher purity; calibration manifold volume: standard values from the manual; calibration sample cell: standard values from the manual; flow degassing: standard values from the manual. Prior to the measurements the samples are degassed at 150 °C for 1 h.

In particular, the following gases have been used: ultra-high purity nitrogen gas from a cylinder or a different source of prepurified nitrogen gas and ultra-high purity helium gas from a cylinder or a different source of prepurified helium gas.

*b) Reduction temperature*

The temperature of reduce catalysts were measured by Temperature Programmed Reduction (TPR). A general description of the method can be found in *'M. Alves Fortunato, D. Aubert, C. Capdeillayre, C. Daniel, A. Hadjar, A. Princivalle, C. Guizard, P. Vernoux, Dispersion measurement of platinum supported on Yttria-Stabilised Zirconia by pulse H2 chemisorption, Applied Catalysis A: General, Volume 403, Issues 1-2, 2011, 18-24'*. This test method is used for the chemical characterization of solid catalysts and to analyze the reduction kinetics of oxide catalyst precursor. It is highly sensitive and does not depend on any specific property of the solid under investigation other than its reducibility. The method is performed by heating the catalyst with a linear temperature ramp in a flow of hydrogen while monitoring the hydrogen consumption. In this way, fingerprint profiles are obtained which allow studying the influence of the support and of promoters on the reducibility. Furthermore, the amount of reducible species in the catalyst and their degree of reduction can be derived from the integrated hydrogen consumption, and lumped kinetic parameters can be estimated if an adequate model of the reduction process exists.

The temperature program reduction (TPR) was carried out by feeding 5% $H_2$ in Ar to 25 mg of catalyst sample in a quartz tube reactor with a flow rate of 30 ml/min. The temperature was raised from room temperature to 950 °C and rate of hydrogen consumption was determined by a thermal conductivity detector (TCD). The device used for this method was a ChemBet Pulsar TPR/TPD using the following parameters: balance: analytical, with 0.1 mg sensitivity; ultra-high purity helium gas from a cylinder or other source of prepurified helium gas, high purity 5% hydrogen in argon gas from a cylinder; high purity oxygen in helium gas from a cylinder; high purity carbon dioxide gas from a cylinder; weight sample cell and adjust flow rate: standard values according to the manual.

*c) Particle size*

The particle size of catalysts was measuring the morphology images by electron microscope (SEM). Furthermore, scanning electron microscopy (SEM, JSM-6301F, JEOL) was used to determine the morphology of the catalysts. The samples are mounted on a stub of metal with adhesive (carbon tap) coated with gold and then studied in the microscope. The described method follows the ASTM standard E986:04 (Standard practice for Scanning Electron Microscope Beam Size Characterization).

Generally it is necessary to apply a conductive layer (carbon or gold) to the sample surface to eliminate charging. In this case, the samples are coated with gold using gold sputter coating machine. Subsequently, the sample is mounted on a stub of metal with adhesive (carbon tap) coated with gold and then observed under the scanning electron microscope (SEM).

When samples are prepared for scanning electron microscopy, a map identifying sample location on an SEM mount can be constructed to assist in locating the sample when performing the analysis.

The following parameters have been used: beam voltage: 20-30 KeV; beam current should be adjusted to yield an X-ray detector dead time of approximately 30 percent; live time: 100-200 s.

*d) Metal loading*

The compositional analysis of the major and minor elements in the catalyst is determined by X-ray fluorescence (XRF) techniques. The described method follows the ASTM standard D4326:04 (Standard Test Method for Major and Minor Elements in Coal and Coke Ash By X-Ray Fluorescence).

The catalyst is ground and pressed into a pellet as a pressed powder specimen. The pellet is then irradiated by an X-ray beam of short wavelength (high energy). The characteristic X-rays of the atom that are emitted or fluoresced upon absorption of the primary or incident X-rays are dispersed and intensities are measured at selected wavelengths by sensitive detectors. The detector output is related to the concentration by calibration curves or by computerized data-handling equipment.

The K spectral lines are used for all of the elements determined by this procedure. All elements are determined as the element and reported as the oxide.

The following devices are used in this method: a compactor, which is a press equipped with a gage enabling reproducible pressures; an excitation source with a stable electrical power supply and a high-intensity, short-wavelength X-ray capability, a spectrometer, which is a wavelength or energy dispersive system equipped with a vacuum sample chamber; a binder, which does not cause spectral interferences during the determination.

Standards for calibration may be prepared from standard reference materials or synthetically blended pure compounds. It is required that the range of concentrations represented by the standards exceeds that of any unknown.

Calculation of elemental concentrations may be accomplished by empirical fundamental parameter or linear regression.

*e) Metal dispersion*

The degree of metal dispersion of the supported metal catalysts is determined by volumetric hydrogen chemisorption. A general description of the method can be found in *'M. Alves Fortunato, D. Aubert, C. Capdeillayre, C. Daniel, A. Hadjar, A. Princivalle, C. Guizard, P. Vernoux, Dispersion measurement of platinum supported on Yttria-Stabilised Zirconia by pulse H2 chemisorption, Applied Catalysis A: General, Volume 403, Issues 1-2, 2011, 18-24'.*

The chemisorption techniques are very well established analytical methods to evaluate the free metal specific surface area and metal dispersion degree. During these methods a chemical reaction between a reactive gas and the catalyst is performed. A common procedure preceding the actual pre-treatment is cleaning the catalyst surface. Cleaning is generally performed by degassing the sample at a suitable temperature to remove water or other vapors eventually adsorbed on the surface. The degree of metal dispersions of the supported metal catalysts is evaluated by measuring the hydrogen adsorption at room temperature.

During the measurement the total hydrogen uptake (volume of hydrogen [$cm^3$], weight of catalyst [g]) is determined. From this data the percent metal dispersion of the supported metal catalysts is calculated.

For the measurement, the chemisorption analyzer ChemBet Pulsar TPR/TPD has been used with the following parameters: balance: analytical, with 0.1 mg sensitivity; high-purity hydrogen gas from a cylinder or a different source of prepurified hydrogen gas; weight sample cell: standard values from manual; flow rate: standard values from manual; degassing conditions: standard values from manual; temperature: room temperature.

The amount of adsorbed hydrogen covering the catalyst surface as a monomolecular layer is obtained by extrapolating the curve relating the amount of hydrogen adsorbed to the adsorption equilibrium pressure of hydrogen to zero.

The metal area is calculated by equation 1:

$$A = \frac{NmXm}{M} \text{ (equation 1)}$$

where Nm is the monolayer coverage at zero pressure expressed in surface atoms per weight metal determined by back extrapolation to zero pressure, M is the number of metal atoms per unit area of crystalline surface and Xm is the chemisorption stoichiometry.

The percentage metal dispersion D is defined as the ratio of the number of the surface atoms to the total number of metal atoms present in the sample. The percentage of the metal dispersion can be calculated from the catalyst composition and the metal surface area by equation 2:

$$D = \frac{AW}{NaX} \text{ (equation 2)}$$

where W is the molecular weight of the metal, N is Avogadro's number, a is the area per surface metal atom, and X is the mass fraction of a metal.

*f) Weight ratios Ag:Cu and MgO:SiO$_2$*

These weight ratios are calculated from the respective metal loadings determined by X-ray fluorescence (XRF) techniques according to ASTM D4326:04.

## 2. Examples

*a) Catalyst preparation (Impregnation)*

A silica ($SiO_2$) and magnesium oxide (MgO) mixture was prepared by wet-kneading of silica ($SiO_2$) and magnesium oxide (MgO) in a weight ratio of 1:2 for 8 h at room temperature. Where only one oxide was used, no wet kneading was applied.

The resulting mixture was dried at 80 °C for 6 hand calcined at 400 °C for 4 h. Silver (Ag) and copper (Cu) were applied as silver chloride and copper chloride by incipient wetness impregnation of the resulting magnesium oxide (MgO) and silica ($SiO_2$) support with an appropriate amount of aqueous metal chloride solutions at 70 °C for 3 h (see Table 1).

The solution was filtrated and the filtrate washed three times with deionized water and another three times with ethanol. Subsequently, the washed filtrate was dried at 100-150 °C and calcined at 300-500 °C for 4 h. Finally, the calcined filtrate was reduced using hydrogen gas at 300-500 °C.

*b) Catalyst preparation (Microwave)*

A silica ($SiO_2$) and magnesium oxide (MgO) mixture was prepared by wet-kneading of silica ($SiO_2$) and magnesium oxide (MgO) in a weight ratio of 1:2 for 8 h at room temperature. The resulting mixture was dried at 80 °C for 6 h and calcined at 400 °C for 4 h. Silver (Ag) and copper (Cu) were applied as silver chloride and copper chloride by incipient wetness impregnation of the resulting magnesium oxide (MgO) and silica ($SiO_2$) support with an appropriate amount of aqueous metal chloride solutions at 70 °C for 3h.

The solution was filtrated and the filtrate washed three times with deionized water and another three times with ethanol. Subsequently, the solution was microwaved for 5 min and calcined at 300-500 °C for 4 h. Finally, the calcined filtrate was reduced using hydrogen gas at 300-500 °C.

*c) Characterization of catalysts prepared by impregnation*

Table 1: Physical properties of catalysts prepared by impregnation.

| | | IE | | | | | | | | | | | | | CE | |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 1 | 2 |
| Support | $SiO_2$ | X | X | X | X | X | X | X | X | X* | X | | | X | X |
| | MgO | X | X | X | X | X | X | X | X | | | X** | X | X | X |
| Metal loading | % | 5 | 5 | 5 | 5 | 5 | 10 | 15 | 20 | 5 | 5 | 5 | 5 | 5 | 5 |
| Ag:Cu [weight ratio] | | 1:1 | 3:1 | 2:1 | 1:2 | 1:3 | 1:1 | 1:1 | 1:1 | 1:1 | 1:1 | 1:1 | 1:1 | 1:0 | 0:1 |
| Surface area $m^2/g$ | | 181 | 165 | 172 | 185 | 189 | 173 | 168 | 152 | 82 | 161 | 71 | 173 | 173 | 176 |
| Reduction Temperature (TPR) | °C | 241 | 240 | 239 | 243 | 243 | 245 | 247 | 250 | 287 | 262 | 274 | 251 | 239 | 245 |
| Particle size | $\mu$m | 20-30 | 20-30 | 20-30 | 20-30 | 20-30 | 20-30 | 20-30 | 20-30 | 40-55 | 20-30 | 40-60 | 20-30 | 20-30 | 20-30 |
| * silica (conventional); all other examples comprise silica fume<br>** magnesium oxide (conventional); all other examples comprise nano-sized magnesium oxide | | | | | | | | | | | | | | | | |

Silica fume, also known as microsilica, (CAS number 112945-52-5) is an amorphous (non-crystalline) polymorph of silicon dioxide (silica). It is an ultrafine powder collected as a by-product of silicon and ferrosilicon alloy production and consists of spherical particles. Silica fume has a particle size of about 7 nm and a surface area of about 370 to 420 $m^2$/g. Nano-sized magnesium oxide (CAS number 1309-48-4) is nano powder, the particle size of which is $\leq 50$ nm.

Increased metal loadings for Ag-Cu catalysts from 5% to 10%, 15%, or 20% result in lower surface areas. The temperatures of the reduced catalysts, however, stay high due to the agglomeration of the metals silver (Ag) and copper (Cu) on the surface of the support (table 1, IE1, IE6-8).

To compare the influence of the type of support and its particle size, also catalysts with different supports have been prepared, such as conventional magnesium oxide (MgO, IE11) and silica ($SiO_2$, IE9) as well as silica fume (IE10) and nano-sized magnesium oxide (IE12). While the latter ones naturally show lower particle sizes, their surface areas are comparable to the mixed $MgO/SiO_2$ supports (161 $m^2$/g and 173 $m^2$/g) (table 1, IE10 and IE12). The conventional MgO only and $SiO_2$ only supports (IE9 and IE11) show significantly lower particle sizes in the range of 71-82 $m^2$/g.

*d) Characterization of catalysts prepared by microwave*

Table 2: Comparison of physical properties of catalysts prepared by impregnation or microwave.

|  |  | IE1 | IE13 |
|---|---|---|---|
| Support | $SiO_2$ | X | X |
|  | MgO | X | X |
| Metal loading | % | 5 | 5 |
| Ag:Cu [weight ratio] |  | 1:1 | 1:1 |
| Preparation method |  | Impregnation | microwave |
| Surface area | $m^2$/g | 181 | 212 |
| Temperature | °C | 241 | 226 |
| Metal dispersion | % | 7.4 | 10.2 |

*e) General catalyst testing procedure*

The catalytic conversion of ethanol to 1,3-butadiene was carried out in a fixed bed stainless steel reactor. The reaction was performed at 350-400 °C under atmospheric pressure. Typically, 1 g of catalyst was used. In order to avoid thermal decomposition of ethanol in the reactor the following procedure was applied: a layer of quartz wool was placed at the bottom of the reactor tube. Subsequently, the catalyst was mixed with silicon carbide (SiC, 200-450 mesh particle size from Aldrich) using a weight ratio of 1:2. Blank tests at reaction temperature without catalyst were conducted. No significant level of conversion was detected during these tests (a small amount of acetaldehyde was observed due to thermal decomposition of ethanol). Ethanol was kept in an evaporator maintained at constant temperature and was introduced into reactors with nitrogen as the carrier gas. Products were analyzed every 20 min by an online gas chromatograph (GC) equipped with a Porapak™ Q column from Sigma Aldrich® and a methanator using a flame ionization detector (FID) and a thermal conductivity detector (TCD). Besides 1,3-butadiene some amounts of ethylene, acetaldehyde, and other $C_3$-$C_4$ oxygenated compounds were found as byproducts. Carbon balance was calculated as total carbon amount in the analyzed products divided by the total amount of added carbon. The carbon balance was generally better than 95%.

*f) Catalytic activity/deactivation depending on the type of the loaded metals*

Table 3: Catalytic activity of the catalysts measured at 350-400 °C using 0.007 ml/min of EtOH in the feed with nitrogen flow rate of 20 ml/min. Catalysts were prepared by impregnation.

| | Reaction time [h] | IE1 | CE1 | CE2 |
|---|---|---|---|---|
| Support | $SiO_2$ | X | X | X |

(continued)

|  | Reaction time [h] | IE1 | CE1 | CE2 |
|---|---|---|---|---|
| MgO | | X | X | X |
| Metal loading        % | | 5 | 5 | 5 |
| Ag:Cu [weight ratio] | | 1:1 | 1:0 | 0:1 |
| Yield | 0 | 52.6 | 57.8 | 48.5 |
| % | 3 | 42.7 | 32.3 | 31.2 |
| | 6 | 38.1 | 20.8 | 26.0 |
| Deactivation | 3 | 18.7 | 44.0 | 35.7 |
| % | 6 | 27.4 | 64.0 | 46.4 |

The catalyst comprising silver (Ag) and copper (Cu) (IE1) according to the invention exhibits higher 1,3-butadiene yields (Table 3) in comparison to CE1 and CE2, which both comprise either only silver (Ag) or copper (Cu).

*g) Catalytic activity/deactivation depending on the particle size of the catalyst support*

Table 4: Catalytic activity depending on the particle size of the catalyst support measured at 350-400 °C using 0.007 ml/min of EtOH in the feed with nitrogen flow rate of 20 ml/min. Catalysts were prepared by impregnation.

|  | Reaction time [h] | IE1 | IE9 | IE10 | IE11 | IE12 |
|---|---|---|---|---|---|---|
| Support        $SiO_2$ | | X | X* | X | | |
| MgO | | X | | | X** | X |
| Metal loading        % | | 5 | 5 | 5 | 5 | 5 |
| Ag:Cu [weight ratio] | | 1:1 | 1:1 | 1:1 | 1:1 | 1:1 |
| Particle size        μm | | 20-30 | 40-55 | 20-30 | 40-60 | 20-30 |
| Yield | 0 | 52.6 | 20.9 | 44.2 | 14.9 | 21.1 |
| % | 6 | 38.1 | 15.2 | 34.9 | 10.2 | 15.9 |
| Deactivation        % | 6 | 27.4 | 27.0 | 21.0 | 31.5 | 24.6 |

Smaller particle sizes of the catalyst support such as silica fume (IE10) and nano-sized magnesium oxide (IE12) result in better performance and stability of the catalyst. A mixture of silica ($SiO_2$) and magnesium oxide (MgO) further enhances these properties (IE1).

*h) Catalytic activity/deactivation depending on preparation method of the catalyst*

Table 5: Catalytic activity of the catalysts measured at 350-400 °C using 0.007 ml/min of EtOH in the feed with a nitrogen flow rate of 20 ml/min.

|  | Reaction time [h] | IE1 | IE13 |
|---|---|---|---|
| Support        $SiO_2$ | | X | X |
| MgO | | X | X |
| Metal loading        % | | 5 | 5 |
| Ag:Cu [weight ratio] | | 1:1 | 1:1 |
| Preparation | | Impregnation | microwave |

(continued)

|  | | Reaction time [h] | IE1 | IE13 |
|---|---|---|---|---|
| Yield | % | 0 | 52.6 | 63.7 |
|  |  | 6 | 38.1 | 50.8 |
| Deactivation | % | 6 | 27.4 | 20.1 |

Preparation by microwave (table 5, IE13) results in higher 1,3-butadiene yields and better stability in comparison to the impregnation method (IE1).

*i) Catalytic activity/deactivation depending on the Ag:Cu weight ratio*

Table 6: Catalytic activity of the different Ag:Cu weight ratios measured at 350-400 °C using 0.007 ml/min of EtOH in the feed with a nitrogen flow rate of 20 ml/min. Catalysts were prepared by either impregnation or microwave.

| | | Reaction time [h] | IE2 | IE3 | IE1 | IE4 | IE5 | IE14 | IE15 | IE13 | IE16 | IE17 |
|---|---|---|---|---|---|---|---|---|---|---|---|---|
| Support | SiO$_2$ | | X | X | X | X | X | X | X | X | X | X |
| | MgO | | X | X | X | X | X | X | X | X | X | X |
| Metal loading | % | | 5 | 5 | 5 | 5 | 5 | 5 | 5 | 5 | 5 | 5 |
| Ag:Cu [weight ratio] | | | 3:1 | 2:1 | 1:1 | 1:2 | 1:3 | 3:1 | 2:1 | 1:1 | 1:2 | 1:3 |
| Preparation method | | | impregnation | | | | | microwave | | | | |
| Yield | % | 0 | 57.9 | 54.8 | 52.6 | 49.4 | 44.3 | 65.6 | 65.4 | 63.7 | 55.9 | 50.5 |
| | | 6 | 22.5 | 26.7 | 38.1 | 36.3 | 34.5 | 39.2 | 42.8 | 50.8 | 45.3 | 42.3 |
| Deactivation | % | 6 | 61.1 | 51.2 | 27.4 | 26.5 | 22.1 | 40.2 | 34.5 | 20.1 | 18.9 | 16.2 |

Increased silver (Ag) content enhances the 1,3-butadiene yield for catalysts prepared by impregnation (IE1-5), but provides less stability of the catalyst. On the other hand, increased copper (Cu) content results in low 1,3-butadiene yields, but stabilizes the impregnation prepared catalysts. The same trend can be recognized for catalysts prepared by the microwave method (IE13-17). However, both activity and stability are enhanced in comparison to the catalyst prepared by impregnation.

*j) Catalytic activity/deactivation depending on the metal loading*

Table 7: Catalytic activity depending on the metal loading of the catalysts measured at 350-400 °C using 0.007 ml/min of EtOH in the feed with a nitrogen flow rate of 20 ml/min. Catalysts were prepared by impregnation.

| | | Reaction time [h] | IE1 | IE6 | IE7 | IE8 | IE18 | IE19 | IE20 | IE21 |
|---|---|---|---|---|---|---|---|---|---|---|
| Support | SiO$_2$ | | X | X | X | X | X | X | X | X |
| | MgO | | X | X | X | X | X | X | X | X |
| Metal loading | % | | 5 | 10 | 15 | 20 | 5 | 10 | 15 | 20 |
| Ag:Cu | | | 1:1 | 1:1 | 1:1 | 1:1 | 1:1 | 1:1 | 1:1 | 1:1 |
| Preparation method | | | impregnation | | | | microwave | | | |
| Yield | % | 0 | 52.6 | 54.7 | 53.9 | 49.8 | 63.7 | 67.8 | 66.3 | 60.7 |
| | | 6 | 38.1 | 35.5 | 31.2 | 26.9 | 50.8 | 53.9 | 48.2 | 43.1 |
| Deactivation | % | 6 | 27.4 | 35.1 | 42.1 | 45.9 | 20.1 | 20.5 | 27.3 | 28.9 |

Metal loadings higher than 5% result in lower 1,3-butadiene yields and thus faster deactivation of the catalyst prepared by the impregnation method (IE1, IE6-8). In case of the microwave prepared catalysts (IE18-21), a metal loading of 10% results in highest 1,3-butadiene yield. Increased metal loading starting from 15% showed a decrease in 1,3-butadiene yields and stability

**Claims**

1. A catalyst for the conversion of ethanol to 1,3-butadiene comprising a support, **characterized in that** silver (Ag) and copper (Cu) are present on the support in metal form,
   wherein the support comprises a first metal oxide, wherein the first metal oxide of the support is silica,
   wherein the support further comprises a second metal oxide, which is different from the first metal oxide, and wherein the second metal oxide is magnesium oxide, and
   wherein the weight ratio between silver (Ag) and copper (Cu) on the support is in the range of 10:1 and 1:10.

2. The catalyst according to claim 1, wherein the silica of the support is silica fume.

3. The catalyst according to claims 1 or 2, wherein the weight ratio between the first and the second metal oxide in the support is in the range of 100:1 to 1:100.

4. The catalyst according to any of the preceding claims, wherein the particle size of the catalyst is between 1 and 100 μm, measured by electron microscopy (SEM) according to ASTM standard E986:04.

5. The catalyst according to any of the preceding claims, wherein the combined weight (metal loading) of Ag and Cu present on the support is in the range of 1% and 30%, based on the total weight of the catalyst, measured by X-ray fluorescence (XRF) techniques according to ASTM standard D4326:04.

6. The catalyst of any of the preceding claims, wherein the surface area of the catalyst is in the range of 60 to 400 m$^2$/g, measured by Brunauer-Emmett-Teller method (BET) according to ASTM standard D6556:10.

7. The catalyst according to any of the preceding claims, wherein the dispersion of Ag and Cu metal on the support is in the range of 2% to 20%, measured by volumetric hydrogen chemisorption.

8. A method for preparing a catalyst according to claim 1 comprising the steps of

   a) calcining a support;
   b) impregnating the calcined support with precursors of silver (Ag) and copper (Cu) metal; and
   c) reducing the impregnated catalyst precursor yielding the catalyst so that silver (Ag) and copper (Cu) in metal form are present on the support in a weight ratio between silver (Ag) and copper (Cu) on the support is in the range of 10:1 and 1:10,

   wherein the support comprises a first metal oxide, wherein the first metal oxide is silica,
   wherein the support further comprises a second metal oxide, which is different from the first metal oxide, and
   wherein the method prior to step a) of calcining a support comprises the steps of

   a') wet-kneading of the first and the second metal oxides to yield an intermediate support,
   b') drying the intermediate support,

   wherein the second metal oxide is magnesium oxide (MgO).

9. The method according to claim 8, wherein the weight ratio between the first and the second metal oxide in the support is in the range of 100:1 to 1:100.

10. The method according to any of claims 8 to 9, further comprising the following steps after impregnation step b) and preceding reduction step c):

    a") filtering the impregnated catalyst precursor;
    b") washing the filtered impregnated catalyst precursor;
    c") drying the washed catalyst precursor; and
    d") calcining the dried catalyst precursor.

11. The method according to claim 8 to 9, further comprising the following steps after impregnation step b) and preceding reduction step c):

    a") filtering the impregnated catalyst precursor;
    b") washing the filtered impregnated catalyst precursor;
    c"') microwaving the washed catalyst precursor;
    d"') calcining the microwaved catalyst precursor.

12. The method according to any of claims 8 to 11, wherein the precursors of Ag and Cu metal are Ag and Cu salts.

13. The method of claim 12 wherein the precursors of Ag and Cu metal are Ag and Cu chlorides or Ag and Cu nitrates.

14. Use of the catalyst according to any of the claims 1 to 7 for the conversion of ethanol to 1,3-butadiene.

15. A process for the catalytic conversion of ethanol to 1,3-butadiene **characterized in that** said process utilizes the catalyst according to any of claims 1 to 7.

**Patentansprüche**

1. Katalysator zur Umwandlung von Ethanol zu 1,3-Butadien, umfassend einen Träger, **dadurch gekennzeichnet, dass** Silber (Ag) und Kupfer (Cu) auf dem Träger in Metallform vorliegen,
   wobei der Träger ein erstes Metalloxid umfasst, wobei das erste Metalloxid von dem Träger Silica ist,
   wobei der Träger ferner ein zweites Metalloxid umfasst, das sich von dem ersten Metalloxid unterscheidet, und
   wobei das zweite Metalloxid Magnesiumoxid ist, und
   wobei das Gewichtsverhältnis zwischen Silber (Ag) und Kupfer (Cu) auf dem Träger in dem Bereich von 10:1 und 1:10 liegt.

**2.** Katalysator nach Anspruch 1, wobei die Silica von dem Träger Silicastaub ist.

**3.** Katalysator nach Anspruch 1 oder 2, wobei das Gewichtsverhältnis zwischen dem ersten und dem zweiten Metalloxid in dem Träger in dem Bereich von 100:1 bis 1:100 liegt.

**4.** Katalysator nach einem der vorhergehenden Ansprüche, wobei die Partikelgröße von dem Katalysator zwischen 1 und 100 $\mu$m liegt, gemessen mittels Elektronenmikroskopie (SEM) gemäß dem ASTM-Standard E986:04.

**5.** Katalysator nach einem der vorhergehenden Ansprüche, wobei das kombinierte Gewicht (Metalllast) von Ag und Cu, die auf dem Träger vorliegen, in dem Bereich von 1 % und 30 % liegt, basierend auf dem Gesamtgewicht des Katalysators, gemessen mittels Techniken der Röntgenfluoreszenz (XRF; für Englisch "X-ray fluorescence") gemäß dem ASTM-Standard D4326:04.

**6.** Katalysator noch einem der vorhergehenden Ansprüche, wobei der Oberflächenbereich von dem Katalysator in dem Bereich von 60 bis 400 m$^2$/g liegt, gemessen mittels dem Brunauer-Emmett-Teller-Verfahren (BET) gemäß dem ASTM-Standard D6556:10.

**7.** Katalysator nach einem der vorhergehenden Ansprüche, wobei die Dispersion von dem Ag- und Cu-Metall auf dem Träger in dem Bereich von 2 % bis 20 % liegt, gemessen mittels volumimetrischer Wasserstoffchemisorption.

**8.** Verfahren zum Herstellen von einem Katalysator nach Anspruch 1, umfassend die folgenden Schritte:

a) Kalzinieren von einem Träger;
b) Imprägnieren von dem kalzinierten Träger mit Vorläufern von Silber-(Ag)- und Kupfer-(Cu)-Metall; und
c) Reduzieren von dem imprägnierten Katalysatorvorläufer, was den Katalysator ergibt, so dass Silber (Ag) und Kupfer (Cu) auf dem Träger in Metallform vorliegen und das Gewichtsverhältnis zwischen Silber (Ag) und Kupfer (Cu) auf dem Träger in dem Bereich von 10:1 und 1:10 liegt,

wobei der Träger ein erstes Metalloxid umfasst, wobei das erste Metalloxid Silica ist,
wobei der Träger ferner ein zweites Metalloxid umfasst, das sich von dem ersten Metalloxid unterscheidet, und
wobei das Verfahren vor dem Schritt a) des Kalzinierens von einem Träger die folgenden Schritte umfasst:

a') Nasskneten von dem ersten und zweiten Metalloxid, um einen Zwischenträger zu erhalten,
b') Trocknen von dem Zwischenträger,

wobei das zweite Metalloxid Magnesiumoxid (MgO) ist.

**9.** Verfahren nach Anspruch 8, wobei das Gewichtsverhältnis zwischen dem ersten und dem zweiten Metalloxid in dem Träger in dem Bereich von 100:1 bis 1:100 liegt.

**10.** Verfahren nach einem der Ansprüche 8 bis 9, ferner umfassend nach dem Imprägnierschritt b) und vor dem Reduktionsschritt c) die folgenden Schritte:

a") Filtern von dem imprägnierten Katalysatorvorläufer;
b") Waschen von dem gefilterten imprägnierten Katalysatorvorläufer;
c") Trocknen von dem gewaschenen Katalysatorvorläufer; und
d") Kalzinieren von dem getrockneten Katalysatorvorläufer.

**11.** Verfahren nach Anspruch 8 bis 9, ferner umfassend nach dem Imprägnierungsschritt b) und vor dem Reduktionsschritt c) die folgenden Schritte:

a") Filtern von dem imprägnierten Katalysatorvorläufer;
b") Waschen von dem gefilterten imprägnierten Katalysatorvorläufer;
c"') Mikrowellenbehandeln von dem gewaschenen Katalysatorvorläufer; und
d"') Kalzinieren von dem mikrowellenbehandelten Katalysatorvorläufer.

**12.** Verfahren nach einem der Ansprüche 8 bis 11, wobei die Vorläufer von dem Ag- und Cu-Metall Ag- und Cu-Salze sind.

**13.** Verfahren nach Anspruch 12, wobei die Vorläufer von dem Ag- und Cu-Metall Ag- und Cu-Chloride oder Ag- und Cu-Nitrate sind.

**14.** Verwendung von dem Katalysator nach einem der Ansprüche 1 bis 7 für die Umwandlung von Ethanol zu 1,3-Butadien.

**15.** Prozess für die katalytische Umwandlung von Ethanol zu 1,3-Butadien, **dadurch gekennzeichnet, dass** der Prozess den Katalysator nach einem der Ansprüche 1 bis 7 verwendet.

## Revendications

**1.** Catalyseur pour la conversion d'éthanol en 1,3-butadiène comprenant un support, **caractérisé en ce que** de l'argent (Ag) et du cuivre (Cu) sont présents sur le support sous forme métallique,
dans lequel le support comprend un premier oxyde métallique, lequel premier oxyde métallique du support est la silice,
dans lequel le support comprend en outre un deuxième oxyde métallique, qui est différent du premier oxyde métallique, lequel deuxième oxyde métallique est l'oxyde de magnésium, et
dans lequel le rapport en poids entre l'argent (Ag) et le cuivre (Cu) sur le support est situé dans la plage allant de 10/1 à 1/10.

**2.** Catalyseur selon la revendication 1, dans lequel la silice du support est de la fumée de silice.

**3.** Catalyseur selon la revendication 1 ou 2, dans lequel le rapport en poids entre les premier et deuxième oxydes métalliques dans le support est situé dans la plage allant de 100/1 à 1/100.

**4.** Catalyseur selon l'une quelconque des revendications précédentes, dans lequel la granulométrie du catalyseur, mesurée par microscopie électronique (MEB) conformément à la norme ASTM E986:04, est comprise entre 1 et 100 $\mu$m.

**5.** Catalyseur selon l'une quelconque des revendications précédentes, dans lequel le poids combiné (charge de métal) de l'Ag et du Cu présents sur le support, mesuré par des techniques de fluorescence aux rayons X (XRF) conformément à la norme ASTM D4326:04, est situé dans la plage allant de 1 % à 30 % par rapport au poids total du catalyseur.

**6.** Catalyseur selon l'une quelconque des revendications précédentes, dans lequel la surface spécifique du catalyseur, mesurée par le procédé de Brunauer-Emmet-Teller (BET) conformément à la norme ASTM D6556:10, est située dans la plage allant de 60 à 400 m$^2$/g.

**7.** Catalyseur selon l'une quelconque des revendications précédentes, dans lequel la dispersion d'Ag et de Cu métalliques sur le support, mesurée par chimisorption d'hydrogène volumétrique, est située dans la plage allant de 2 % à 20 %.

**8.** Procédé pour préparer un catalyseur selon la revendication 1, comprenant les étapes suivantes :

a) calcination d'un support ;
b) imprégnation du support calciné avec des précurseurs d'argent (Ag) et de cuivre (Cu) métalliques ; et
c) réduction du précurseur de catalyseur imprégné, ce qui engendre le catalyseur de façon que de l'argent (Ag) et du cuivre (Cu) sous forme métallique soient présents sur le support en un rapport en poids entre l'argent (Ag) et le cuivre (Cu) situé dans la plage allant de 10/1 à 1/10,

dans lequel le support comprend un premier oxyde métallique, lequel premier oxyde métallique est la silice,
dans lequel le support comprend en outre un deuxième oxyde métallique, qui est différent du premier oxyde métallique, et
lequel procédé, avant l'étape a) de calcination du support, comprend les étapes suivantes :

a') malaxage à l'état humide des premier et deuxième oxydes métalliques pour engendrer un support intermédiaire,
b') séchage du support intermédiaire,

17

dans lequel le deuxième oxyde métallique est l'oxyde de magnésium (MgO).

9. Procédé selon la revendication 8, dans lequel le rapport en poids entre les premier et deuxième oxydes métalliques dans le support est situé dans la plage allant de 100/1 à 1/100.

10. Procédé selon l'une quelconque des revendications 8 et 9, comprenant en outre les étapes suivantes après l'étape d'imprégnation b) et avant l'étape de réduction c)

   a") la filtration du précurseur de catalyseur imprégné ;
   b") le lavage du précurseur de catalyseur imprégné filtré ;
   c") le séchage du précurseur de catalyseur lavé ; et
   d") la calcination du précurseur de catalyseur séché.

11. Procédé selon la revendication 8 ou 9, comprenant en outre les étapes suivantes après l'étape d'imprégnation b) et avant l'étape de réduction c) :

   a") la filtration du précurseur de catalyseur imprégné ;
   b") le lavage du précurseur de catalyseur imprégné filtré ;
   c") le passage au micro-ondes du précurseur de catalyseur lavé ;
   d") la calcination du précurseur de catalyseur passé aux micro-ondes.

12. Procédé selon l'une quelconque des revendications 8 à 11, dans lequel les précurseurs d'Ag et de Cu métalliques sont des sels d'Ag et de Cu.

13. Procédé selon la revendication 12, dans lequel les précurseurs d'Ag et de Cu métalliques sont des chlorures d'Ag et de Cu ou des nitrates d'Ag et de Cu.

14. Utilisation du catalyseur selon l'une quelconque des revendications 1 à 7 pour la conversion d'éthanol en 1,3-butadiène.

15. Procédé pour la conversion catalytique d'éthanol en 1,3-butadiène, **caractérisé en ce que** ledit procédé utilise le catalyseur selon l'une quelconque des revendications 1 à 7.

## REFERENCES CITED IN THE DESCRIPTION

**Patent documents cited in the description**

- WO 2012015340 A1

- KR 2014050531 A

**Non-patent literature cited in the description**

- **MAKSHINA ; W. JANSSENS ; B.F. SELS ; P.A. JACOBS.** Catalytic study of the conversion of ethanol into 1,3-butadiene. *Catalysis Today,* 2012, vol. 198, 338-344

- **M. ALVES FORTUNATO ; D. AUBERT ; C. CAPDEILLAYRE ; C. DANIEL ; A. HADJAR ; A. PRINCIVALLE ; C. GUIZARD ; P. VERNOUX.** Dispersion measurement of platinum supported on Yttria-Stabilised Zirconia by pulse H2 chemisorption. *Applied Catalysis A: General,* 2011, vol. 403 (1-2), 18-24